# EUROPEAN PATENT APPLICATION

(11) **EP 4 492 390 A1**
(43) Date of publication of application: **15.01.2025**
(21) Application number: 23788340.0
(22) Date of filing: 11.04.2023
(51) Int. Cl.: G16H 10/20, G16H 70/40

(54) **PROGNOSIS REPORT COLLECTING ASSISTANCE DEVICE, METHOD, PROGRAM, AND COMPUTER-READABLE STORAGE MEDIUM**

(30) Priority: 11.04.2022 JP 2022065230
(71) Applicant: NTT Communications Corporation, Tokyo 100-8019 (JP)
(72) Inventor: TSUNEKAWA, Satoshi, Tokyo 100-8019 (JP); SAKURAI, Yoichi, Tokyo 100-8019 (JP); KONAGAYA, Takahito, Tokyo 100-8019 (JP); KOKUBO, Ryuta, Tokyo 100-8019 (JP)
(74) Representative: Nederlandsch Octrooibureau
(86) International application number: PCT/JP2023/014774
(87) International publication number: WO 2023/199922

(57) **Abstract**

A patient reported outcome collection support apparatus according to one aspect of this invention acquires clinical trial protocol information including a plurality of survey items defined for each clinical trial in advance from an information processing apparatus used by a clinical trial operator of a medicine via a network, generates survey form information used to survey a prognosis of the patient by converting the plurality of survey items included in the acquired clinical trial protocol information based on a conversion rule stored in advance, distributes the generated survey form information to the terminal apparatus of the patient via the network, receives patient reported outcome information indicating an answer result of the patient to the survey form information from the terminal apparatus via the network, and transfers the received patient reported outcome information to the information processing apparatus of the clinical trial operator via the network.

## Description

### FIELD

Embodiments described herein relate generally to a patient reported outcome collection support apparatus that, for example, supports a work of collecting patient reported outcome information from a patient who has taken a medicine after sale-of the medicine by a pharmaceutical company, a method, a program, and a computer-readable storage medium.

### BACKGROUND

In the medical and pharmaceutical fields, a so-called clinical trial for conducting a follow-up survey of prognosis of patients after medication is performed. Conventional clinical trials are performed for, for example, inpatients or outpatients, and in general, a clinical trial coordinator extracts clinical trial object persons based on the medical charts of the patients. Hence, the work of extracting the clinical trial object persons takes labor and time, and a patient who should be extracted as a target is readily omitted.

There has been proposed a system in which the prescription and dispensing information of each inpatient or outpatient are registered in a database, and the registered prescription and dispensing information are collated with clinical trial information representing clinical trial standards set in advance by an information processing apparatus, thereby screening clinical trial object persons and constructing a database of patients adaptive to the clinical trial (for example, see patent literature 1). According to this system, it is possible to efficiently select patients suitable for the clinical trial.

### CITATION LIST

### PATENT LITERATURE

Patent Document 1: Japanese Patent No. 6338957

### SUMMARY

### TECHNICAL PROBLEM

However, when collecting a patient reported outcome (PRO) representing a state after medication from a clinical trial target patient, currently, as described in, for example, patent literature 1, a clinical trial operator in a pharmaceutical company or the like creates a survey form for each clinical trial and asks a cooperator in a medical institution, a pharmacy, or the like for a survey, and the asked cooperator presents the survey form to a clinical trial target patient and causes him/her to enter it, or makes an interview based on the survey form, thereby collecting a PRO. For this reason, the load on the clinical trial operator and the cooperator is large, and collecting PROs requires much labor and time, resulting in a major obstacle in increasing the efficiency of the clinical trial.

The present invention has been made in consideration of the above-described situation, and provides a technique of decreasing labor and time necessary for collecting patient reported outcome information from a patient after medication, thereby improving the efficiency of a clinical trial.

### SOLUTION TO PROBLEM

In order to solve the above-described problem, according to one aspect of a patient reported outcome collection support apparatus or support method of the present invention, in a patient reported outcome collection support apparatus capable of transmitting, via a network, information data between an information processing apparatus used by a clinical trial operator of a medicine and each of a plurality of terminal apparatuses used by clinical trial target patients, protocol information including a plurality of survey items defined for each clinical trial in advance is acquired from the information processing apparatus via the network, and survey form information used to survey a prognosis of the patient is generated by converting the plurality of survey items included in the acquired clinical trial protocol information based on a conversion rule stored in advance. Then, the generated survey form information is distributed to the terminal apparatus via the network, patient reported outcome information of the patient to the survey form information is acquired from the terminal apparatus via the network, and the acquired patient reported outcome information is transferred to the information processing apparatus via the network.

According to one aspect of the present invention, for example, if a person in charge of a clinical trial in a pharmaceutical company or the like transmits, for each clinical trial, protocol information including a plurality of survey items necessary for the clinical trial from the information processing apparatus to the patient reported outcome collection support apparatus, the patient reported outcome collection support apparatus generates survey form information in which questions and answer candidates corresponding to the plurality of survey items included in the protocol information are described. For this reason, the person in charge of the clinical trial need not create the survey form information for each clinical trial by himself/herself, and this can reduce load associated with a preparation work before the start of the clinical trial.

Also, the generated survey form information is distributed from the patient reported outcome collection support apparatus to the terminal apparatus of an adequate patient, and patient reported outcome information of the patient to the survey form information is temporarily collected by the patient reported outcome collection support apparatus and then transferred to the information processing apparatus of the clinical trial operator. For this reason, the person in charge of the clinical trial need not perform the work of distributing the survey form information to the patient and collecting the patient reported outcome information by himself/herself, and this can reduce the work load.

That is, a series of processes from creation of survey form information for a clinical trial of a medicine to collection of patient reported outcome information of the patient is automatically performed by the patient reported outcome collection support apparatus, and this can greatly reduce the work load concerning the clinical trial by the clinical trial operator such as the pharmaceutical company.

### ADVANTAGEOUS EFFECTS OF INVENTION

According to one aspect of the present invention, it is possible to provide a technique of decreasing labor and time necessary for collecting patient reported outcome information from a patient after medication, thereby improving the efficiency of a clinical trial.

### BRIEF DESCRIPTION OF THE DRAWINGS

FIG. 1 is a schematic view of a patient reported outcome information collection system including a server apparatus functioning as a patient reported outcome collection support apparatus according to an embodiment of the present invention.
FIG. 2 is a view showing an example of the functional configuration of the system shown in FIG. 1.
FIG. 3 is a block diagram showing the hardware configuration of the server apparatus shown in FIGS. 1 and 2.
FIG. 4 is a block diagram showing the software configuration of the server apparatus shown in FIGS. 1 and 2.
FIG. 5 is a flowchart showing the first half portion of the processing procedure and processing contents of patient reported outcome collection support processing executed by the server apparatus shown in FIG. 4.
FIG. 6 is a flowchart showing the second half portion of the processing procedure and processing contents of patient reported outcome collection support processing executed by the server apparatus shown in FIG. 4.
FIG. 7 is a view showing an example of the configuration of a setting sheet of survey items and the like provided from, for example, a pharmaceutical company.
FIG. 8 is a view showing an example of the configuration of the upper layer of a test input screen generated based on the setting sheet shown in FIG. 7.
FIG. 9A is a view showing an example of the configuration of the middle layer of the test input screen generated based on the setting sheet shown in FIG. 7.
FIG. 9B is a view showing an example of the configuration of the lower layer of the test input screen generated based on the setting sheet shown in FIG. 7.
FIG. 10A is a view showing an example of the entry rules of an item group/item sheet of the setting sheet shown in FIG. 7.
FIG. 10B is a view showing an example of the entry rules of the item group/item sheet of the setting sheet shown in FIG. 7, continued from FIG. 10A.
FIG. 10C is a view showing an example of the entry rules of the item group/item sheet of the setting sheet shown in FIG. 7, continued from FIG. 10B.
FIG. 10D is a view showing an example of the entry rules of the item group/item sheet of the setting sheet shown in FIG. 7, continued from FIG. 10C.
FIG. 11 is a view showing an example of the entry rules of a code list sheet of the setting sheet shown in FIG. 7.
FIG. 12A is a view showing an example of the entry rules of a Visit sheet of the setting sheet shown in FIG. 7.
FIG. 12B is a view showing an example of the entry rules of the Visit sheet of the setting sheet shown in FIG. 7, continued from FIG. 12A.
FIG. 13 is a view showing an example of the entry rules of a page configuration sheet of the setting sheet shown in FIG. 7.
FIG. 14 is a view for explaining the contents of the page configuration sheet shown in FIG. 13.
FIG. 15 is a view showing the association between the item group/item shown in FIGS. 10A, 10B, 10C, and 10D and the test input screen shown in FIG. 8.
FIG. 16 is a view showing the association between the item group/item shown in FIGS. 10A, 10B, 10C, and 10D and the test input screen shown in FIG. 8.
FIG. 17 is a view showing the association between the item group/item shown in FIGS. 10A, 10B, 10C, and 10D and the test input screen shown in FIGS. 9A and 9B.
FIG. 18 is a view showing the association between the item group/item shown in FIGS. 10A, 10B, 10C, and 10D and the test input screen shown in FIGS. 9A and 9B.
FIG. 19 is a view showing the association between the item group/item shown in FIGS. 10A, 10B, 10C, and 10D and the test input screen shown in FIGS. 9A and 9B.
FIG. 20 is a view showing the association between the item group/item shown in FIGS. 10A, 10B, 10C, and 10D and the test input screen shown in FIGS. 9A and 9B.
FIG. 21 is a view showing the association between the item group/item shown in FIGS. 10A, 10B, 10C, and 10D and the test input screen shown in FIGS. 9A and 9B.
FIG. 22 is a view showing the association between the item group/item shown in FIGS. 10A, 10B, 10C, and 10D and the test input screen shown in FIGS. 9A and 9B.
FIG. 23 is a view showing the association between the item group/item shown in FIGS. 10A, 10B, 10C, and 10D and the test input screen shown in FIGS. 9A and 9B.
FIG. 24 is a view showing the association between the item group/item shown in FIGS. 10A, 10B, 10C, and 10D and the test input screen shown in FIGS. 9A and 9B.
FIG. 25 is a view showing an example of a screen used to input a body temperature in the test input screen shown in FIGS. 9A and 9B.
FIG. 26 is a view showing an example of a screen used to input the symptom at an injection site in the test input screen shown in FIGS. 9A and 9B.
FIG. 27 is a view showing an example of an input screen for an additional description field in the test input screen shown in FIGS. 9A and 9B.
FIG. 28 is a view showing an example of an input result of various kinds of items in the test input screen shown in FIGS. 9A and 9B.
FIG. 29 is a view showing an example of an input result of various kinds of items in the test input screen shown in FIGS. 9A and 9B.

### DETAILED DESCRIPTION

An embodiment of the present invention will now be described with reference to the accompanying drawings.

### [One Embodiment]

### (Configuration Example)

### (1) System

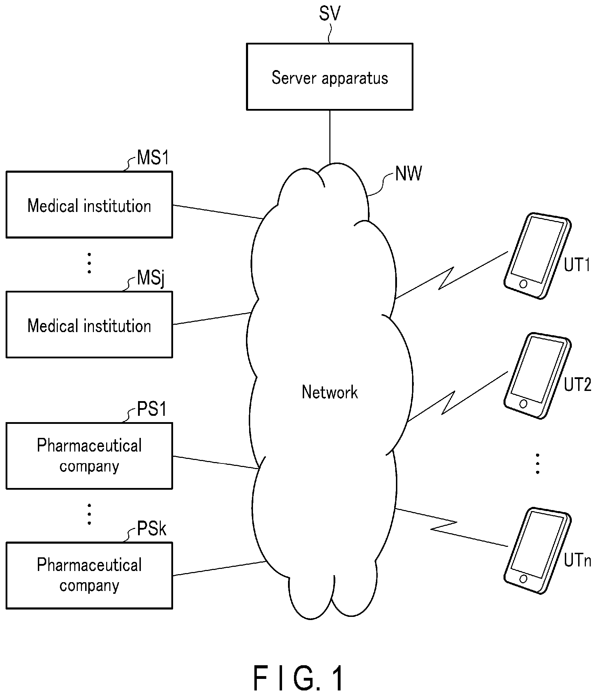

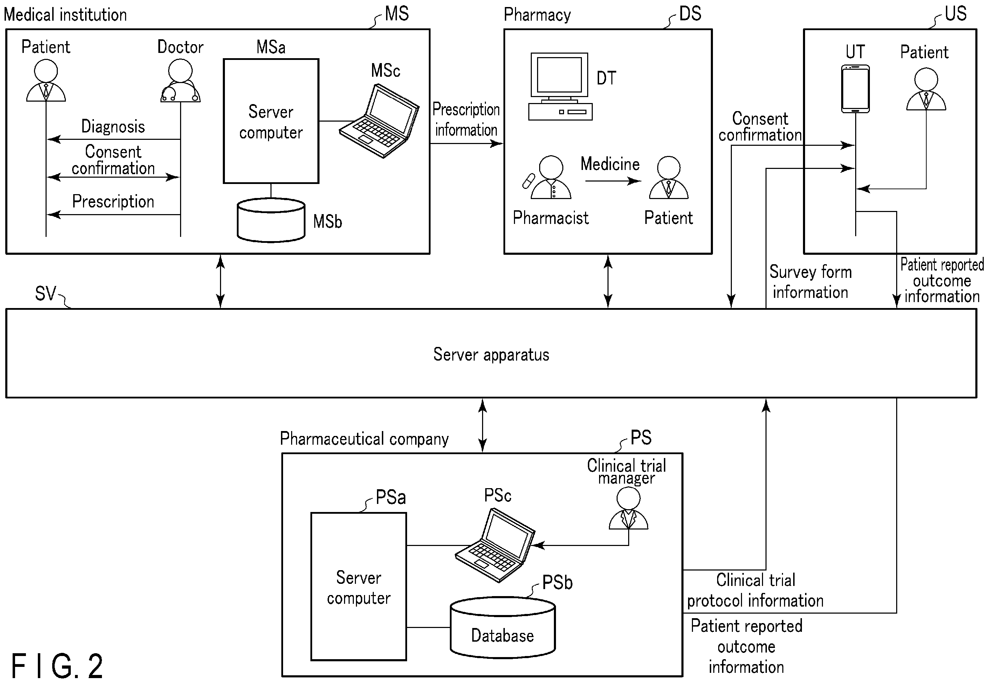

FIG. 1 is a schematic view of a patient reported outcome information collection system including a server apparatus SV functioning as a patient reported outcome collection support apparatus according to an embodiment of the present invention, and FIG. 2 is a view showing an example of the functional configuration of the patient reported outcome information collection.

The server apparatus SV is formed by a server computer arranged on, for example, a web or a cloud. Note that if a multipurpose information processing platform is constructed on the cloud, the server apparatus SV may be provided as some or all of information processing apparatuses constructing the platform.

The server apparatus SV can perform data transmission, via a network NW, between information processing systems operated by a plurality of medical institutions MS1 to MSj (to be collectively referred to as MS hereinafter), information processing systems operated by a plurality of pharmaceutical companies PS1 to PSk (to be collectively referred to as PS hereinafter), and patient terminals UT1 to UTn (to be collectively referred to as UT hereinafter) used by a plurality of patients US1 to USn (to be collectively referred to as US hereinafter). Note that if not only the medical institutions but also pharmacies DS1 to DSi (to be collectively referred to as DS hereinafter) are entrusted to sell a medicine to be subjected to a clinical trial, the server apparatus SV can perform data transmission with the pharmacies DS as well.

The information processing systems of the medical institution MS and the pharmacy DS include server computers MSa and PSa with databases MSb and PSb, and terminals MSc and PSc used by persons in charge, respectively. In the database MSb of the information processing system of the medical institution MS, the attribute information, medical treatment information, and prescription information of a patient are stored for each patient in association with its identification information (to be referred to as a patient ID hereinafter). In the attribute information of a patient, for example, the telephone number or address information of the patient terminal UT used by the patient US is included as the contact information of the patient, in addition to the name, the age, the sex, and the address of the patient.

In the database PSb of the information processing system of the pharmaceutical company PS, medicine attribute information such as the name, components, effects, contraindications, and the like of the medicine is stored for each medicine put on the market in association with its identification information (to be referred to as a medicine ID hereinafter). In the database PSb of the information processing system of the pharmaceutical company PS, patient reported outcome information collected from the patient US via the server apparatus SV in the clinical trial process is also stored.

The patient terminal UT is formed by, for example, a smartphone and includes a browser configured to access an information site including the server apparatus SV, and an application configured to transmit/receive information data to/from the server apparatus SV or another terminal using email, a social network system (SNS), or a short message service (SMS). Note that as the patient terminal UT, a tablet terminal, or a laptop or desktop type personal computer may be used if it has the same function.

The network NW includes, for example, a wide area network including the Internet as a core, and an access network used to access the wide area network. As the access network, for example, a public communication network using a wires or wireless network, a local area network (LAN) using a wires or wireless network, and a cable television (CATV) network are used.

### (2) Server Apparatus SV

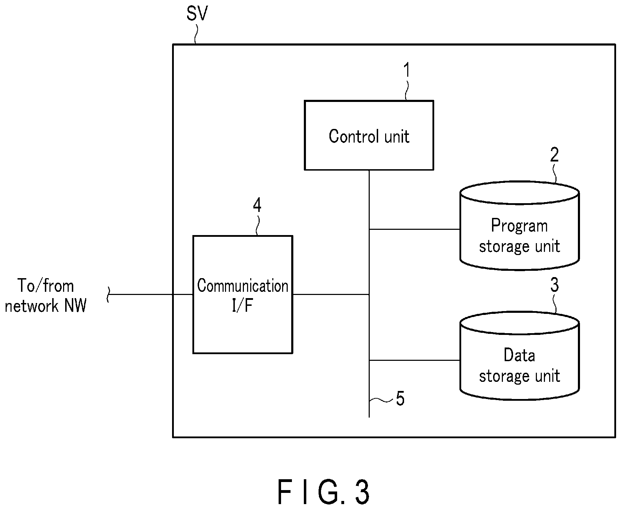

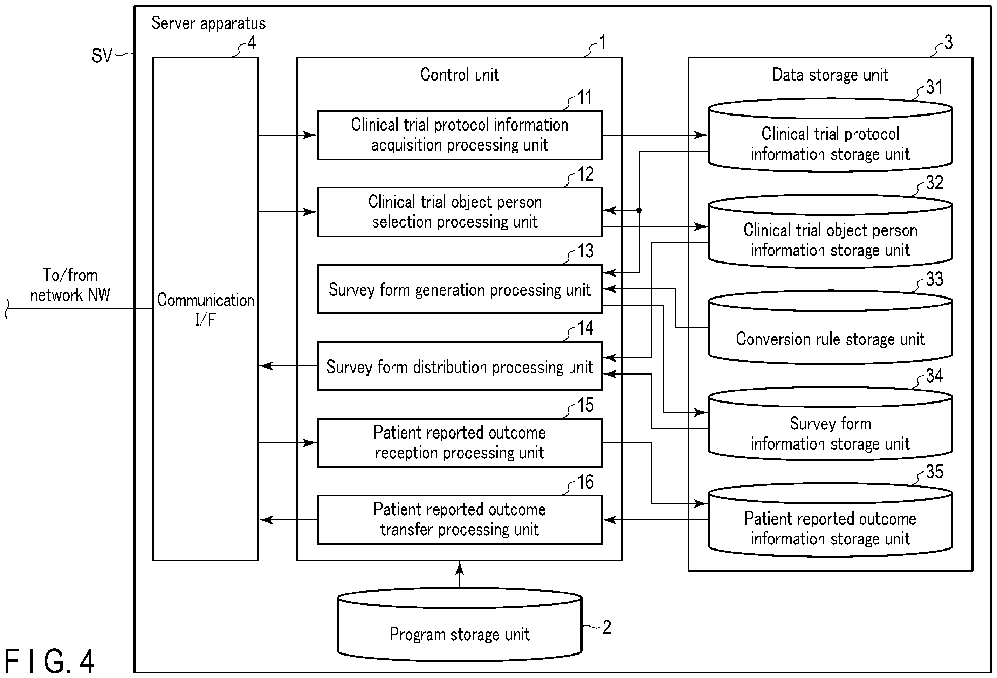

FIGS. 3 and 4 are block diagrams showing the hardware configuration and the software configuration of the server apparatus SV, respectively.

The server apparatus SV includes a control unit 1 using a hardware processor such as a central processing unit (CPU). A storage unit including a program storage unit 2 and a data storage unit 3 and a communication interface (the interface will be referred to as I/F hereinafter) 4 are connected to the control unit 1 via a bus 5.

Under the control of the control unit 1, the communication I/F 4 performs data transmission between the information processing system of the medical institution MS and the information processing system of the pharmaceutical company PS and also the patient terminal UT using a communication protocol defined by the network NW.

The program storage unit 2 is formed, as a storage medium, by combining an occasionally writable and readable nonvolatile memory such as a solid state drive (SSD) and a nonvolatile memory such as a read only memory (ROM), and stores application programs necessary for executing various kinds of processing according to an embodiment in addition to middleware such as an operating system (OS). The OS and the application programs will collectively be referred to as programs hereinafter. Note that the application programs may be stored in the program storage unit 2 in advance, but may be, for example, downloaded from another server apparatus or the like and stored in the program storage unit 2 as needed.

The data storage unit 3 is formed, for example, as a storage medium, by combining an occasionally writable and readable nonvolatile memory such as an SSD and a volatile memory such as a random access memory (RAM), and includes, as main data storage units necessary for executing one embodiment, a clinical trial protocol information storage unit 31, a clinical trial object person information storage unit 32, a conversion rule storage unit 33, a survey form information storage unit 34, and a patient reported outcome information storage unit 35.

The clinical trial protocol information storage unit 31 is used to store clinical trial protocol information sent from the information processing system of the pharmaceutical company PS together with a clinical trial cooperation request. The clinical trial protocol information includes a medicine ID for identifying the medicine as the clinical trial target, list data of survey items of a clinical trial, and information indicating clinical trial conditions, and examples thereof will be described in an operation example.

The clinical trial object person information storage unit 32 is used to store the attribute information of a plurality of patients adaptive to the clinical trial (to be also referred to as clinical trial target patients hereinafter) selected for each clinical trial in association with a clinical trial ID.

The conversion rule storage unit 33 stores conversion rules for generating survey form information for each assumed clinical trial. The conversion rules are used to convert the list data of the survey items included in the clinical trial protocol information into survey form information to be presented to a clinical trial target patient.

The survey form information storage unit 34 is used to store the generated survey form information in association with the clinical trial ID.

The patient reported outcome information storage unit 35 is used to store patient reported outcome information of a patient sent from the patient terminal UT association with the clinical trial ID.

The control unit 1 includes, as processing functions necessary for executing one embodiment, a clinical trial protocol information acquisition processing unit 11, a clinical trial object person selection processing unit 12, a survey form generation processing unit 13, a survey form distribution processing unit 14, a patient reported outcome reception processing unit 15, and a patient reported outcome transfer processing unit 16. All the processing units 11 to 16 are implemented by causing the hardware processor of the control unit 1 to execute the pogroms stored in the program storage unit 2.

The clinical trial protocol information acquisition processing unit 11 performs processing of receiving, via the communication I/F 4, clinical trial protocol information sent from the information processing system of the pharmaceutical company PS together with a clinical trial execution request and storing the received clinical trial protocol information in the clinical trial protocol information storage unit 31 in association with the clinical trial ID.

The clinical trial object person selection processing unit 12 performs processing of selecting, for each clinical trial, a patient adaptive to clinical trial conditions included in the clinical trial protocol information. This selection processing is performed by, for example, referring to the attribute information of a plurality of patients US stored in the database MSb of the information processing system of the medical institution MS and medical treatment information and prescription information included in electronic medical charts. The attribute information of each selected patient adaptive to the clinical trial conditions is then stored as clinical trial object person information in the clinical trial object person information storage unit 32 in association with the clinical trial ID.

The survey form generation processing unit 13 performs processing of importing the list data of the survey items included in the clinical trial protocol information from the clinical trial protocol information storage unit 31 and converting a predetermined format using the conversion rules stored in the conversion rule storage unit 33 for each clinical trial, thereby generating survey form information used to survey the prognosis of a patient. The survey form generation processing unit 13 then temporarily stores the generated survey form information in the survey form information storage unit 34 in association with the clinical trial ID.

The survey form distribution processing unit 14 reads out survey form information stored in the survey form information storage unit 34 for each clinical trial. The survey form distribution processing unit 14 then performs processing of distributing the readout survey form information from the communication I/F 4 to the corresponding patient terminal UT based on the attribute information of the clinical trial target patient stored in the clinical trial object person information storage unit 32.

The patient reported outcome reception processing unit 15 performs processing of receiving, via the communication I/F 4, patient reported outcome information returned from the patient terminal UT that is the distribution destination of the survey form information and storing the received patient reported outcome information in the patient reported outcome information storage unit 35 in association with the clinical trial ID.

The patient reported outcome transfer processing unit 16 performs processing of reading out each or a predetermined number of undistributed patient reported outcome information among the patient reported outcome information stored in the patient reported outcome information storage unit 35, and transferring the readout patient reported outcome information from the communication I/F 4 to the information processing system of the pharmaceutical company PS that is the clinical trial execution request source.

### (Operation Example)

An example of the operation of the server apparatus SV having the above-described configuration will be described next.

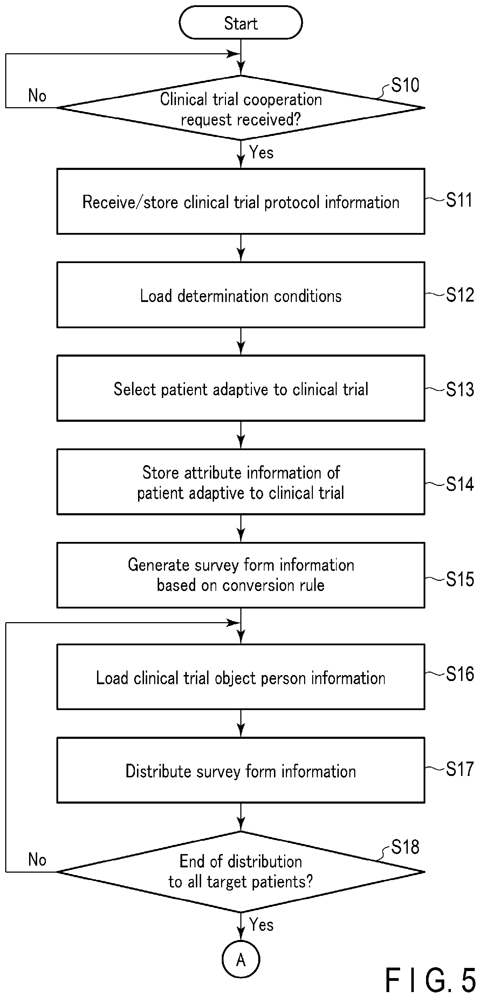

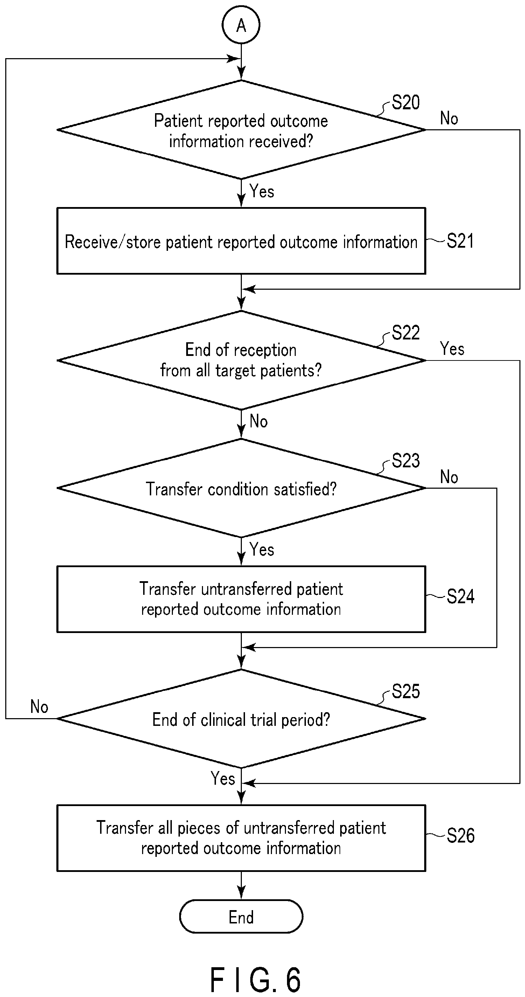

FIGS. 5 and 6 are flowcharts showing the processing procedure and processing contents of patient reported outcome collection support processing executed by the server apparatus SV.

### (1) Acquisition of Clinical Trial Protocol Information

Clinical trial protocol information is created by the information processing system of a pharmaceutical company for each clinical trial in accordance with the type of medicine and the specifications of a clinical trial designated by the clinical trial manager of the pharmaceutical company PS. The clinical trial protocol information includes, for example, the medicine ID for identifying the medicine of the clinical trial target, the list data of the survey items of the clinical trial, and information indicating clinical trial conditions.

The list data of survey items necessary for a clinical trial is described in a data file of a table format using spreadsheet software such as Excel°. In a case of, for example, a vaccine for new type pneumonia, survey items are "fever", "redness", and "pain".

Clinical trial conditions include, for example, the age range, the sex, the residential area, and the clinical trial period of a clinical trial target patient. Note that the survey items and the clinical trial conditions are not limited to the above-described examples, and only some of these may be used, or other items and conditions may be added.

When a pharmaceutical company executes a clinical trial, for example, a clinical trial manager of the pharmaceutical company transmits the clinical trial protocol information from the management terminal PSc to the server apparatus SV together with a clinical trial cooperation request. Note that the transmission of the clinical trial cooperation request and the clinical trial protocol information may automatically be done at a timing designated by the information processing system of the pharmaceutical company PS in advance.

In step S10, the control unit 1 of the server apparatus SV monitors reception of the clinical trial cooperation request under the control of the clinical trial protocol information acquisition processing unit 11. If the clinical trial cooperation request is received from the pharmaceutical company PS in this state, in step S11, the clinical trial protocol information acquisition processing unit 11 subsequently receives the clinical trial protocol information transmitted from the management terminal PSc of the pharmaceutical company PS, and stores the received clinical trial protocol information in the clinical trial protocol information storage unit 31 in association with the clinical trial ID.

### (2) Selection of Clinical Trial Target Patient

Upon receiving the clinical trial cooperation request, first, the control unit 1 of the server apparatus SV performs processing of selecting a clinical trial target patient in the following way under the control of the clinical trial object person selection processing unit 12.

That is, first, in step S12, the clinical trial object person selection processing unit 12 loads clinical trial conditions included in the clinical trial protocol information from the clinical trial protocol information storage unit 31. Next, in step S13, the clinical trial object person selection processing unit 12 refers to medical treatment information and prescription information described in the electronic medical chart of the patient US stored in the database MSb of each medical institution MS and the attribute information of the patient US, and selects, from the plurality of patients US taking the medicine of the clinical trial target, a patient who satisfies the conditions such as the age range, the sex, and the residential area defined by the clinical trial conditions as a clinical trial object person.

In step S14, the clinical trial object person selection processing unit 12 acquires the attribute information of the selected patient from the database, and stores the acquired attribute information of the patient in the clinical trial object person information storage unit 32 in association with a test ID. In this case, however, from the viewpoint of protecting the personal information of the patient, filtering processing is preferably performed to acquire only minimum information necessary for the clinical trial, for example, the age, the sex, and the contact information among information elements included in the attribute information of the patient.

Note that the clinical trial target patient selection processing may be performed by referring to the attribute information of patients acquired from a terminal DT of the pharmacy DS that has sold the medicine of the clinical trial target in place of or in addition to the database MSb of the medical institution MS.

Also, when selecting clinical trial target patients, if the clinical trial conditions include, for example, a request of confirming the consent of each patient to the clinical trial, patients whose consent has already been confirmed are subjected to clinical trial object person selection. The confirmation of consent may be done by a doctor in charge or a pharmacist in the medical institution MS or the pharmacy DS in advance, or may be done between the server apparatus SV and the patient terminal UT upon receiving the clinical trial cooperation request from the pharmaceutical company PS.

If the medical institution MS or the pharmacy DS confirms the consent of a patient to the clinical trial, the consent confirmation result is managed by an electronic medical chart or medicine sale management data. Accordingly, when performing clinical trial object person selection processing, the server apparatus SV can refer to the electronic medical chart or the medicine sale management data of a patient, thereby determining whether the consent has already been confirmed.

Note that an example of processing of executing the consent confirmation procedure between the server apparatus SV and the patient terminal UT will be described concerning survey form information distribution processing to be described later.

### (3) Generation of Survey Form Information

In step S15, if the clinical trial protocol information is acquired, the control unit 1 of the server apparatus SV executes processing of generating survey form information in the following way under the control of the survey form generation processing unit 13.

That is, for example, first, in step S151, the survey form generation processing unit 13 selects, based on the clinical trial ID included in the clinical trial protocol information, a conversion rule corresponding to the clinical trial ID from a plurality of conversion rules stored in the conversion rule storage unit 33.

Next, in step S152, the survey form generation processing unit 13 imports the list data of survey items included in the clinical trial protocol information from the clinical trial protocol information storage unit 31 and converts it into data of a document format using the selected conversion rule. For example, in a case where the list data of the survey items is described in a data file of a table format using spreadsheet software such as Excel, the survey form generation processing unit 13 imports the data file of the table format and converts it into data of a document format.

The survey form generation processing unit 13 then generates, for example, a question text and an answer field in correspondence with each of the plurality of survey items converted into the data of the document format, and generates survey form information in which the generated question texts and answer fields corresponding to the survey items are rearranged in a predetermined order. The survey form generation processing unit 13 stores the generated survey form information in the survey form information storage unit 34 in association with the clinical trial ID. The survey form information is sent to a patient terminal and used as display data for displaying a clinical trial input screen on the display unit of the patient terminal.

### (4) Distribution of Survey Form Information

In a clinical trial period defined in the clinical trial conditions included in the clinical trial protocol information, the control unit 1 of the server apparatus SV distributes the survey form information to each clinical trial target patient in the following way under the control of the survey form distribution processing unit 14.

That is, first, in step S16, the survey form distribution processing unit 14 loads the attribute information of each clinical trial target patient from the clinical trial object person information storage unit 32. The survey form distribution processing unit 14 then determines whether the clinical trial target patient has already confirmed the consent. As a result of the determination, if consent confirmation is not made yet, the survey form distribution processing unit 14 executes the consent confirmation procedure with the patient terminal UT.

As the consent confirmation procedure, various procedures can be considered.

As one of the procedures, the server apparatus SV transmits a consent confirmation request to the patient terminal UT. This is performed, for example, in the following way.

That is, the server apparatus SV first executes a patient authentication procedure with the patient terminal UT. If the identity of the patient is confirmed by the authentication procedure, the server apparatus SV then transmits request information for consent confirmation to the patient terminal UT by mail or the like. At this time, in the request information, for example, the uniform resource locator (URL) of the consent confirmation page of the server apparatus SV is described. If the patient who has received the request information performs an access operation to the URL, the consent confirmation page is downloaded from the server apparatus SV to the patient terminal UT. To consent to the clinical trial, the patient inputs required items in accordance with the guidance of the consent confirmation page and presses an "agree button". On the other hand, not to consent to the clinical trial, the patient presses a "not agree button". The server apparatus SV receives information indicating the consent confirmation result input in the consent confirmation page from the patient terminal UT.

As another consent confirmation procedure, for example, the medical institution MS or the pharmacy DS may request the patient US to confirm the consent to the clinical trial.

That is, when prescribing the medicine to the patient US or selling the medicine to the patient US, the medical institution MS or the pharmacy DS issues a two-dimensional code such as a QR code^{®} or a barcode for consent confirmation to the clinical trial. The two-dimensional code includes a URL used to access the consent confirmation page operated by the server apparatus SV. To consent to the clinical trial, the patient US accesses the two-dimensional code from the patient terminal UT. Then, the patient is temporarily registered in the server apparatus SV. Next, when the patient US performs a consent confirmation operation in the consent confirmation page and inputs contact information such as his/her telephone number or mail address, formal registration is completed.

If the consent confirmation of the clinical trial target patient to the clinical trial is acquired, next, in step S17, the survey form distribution processing unit 14 reads out the survey form information from the survey form information storage unit 34. In accordance with the contact information stored in the clinical trial object person information storage unit 32, the survey form distribution processing unit 14 then transmits the readout survey form information from the communication I/F 4 to the patient terminal UT using, for example, email, SMS, or SNS.

Also, every time survey form information transmission to one patient is ended, in step S18, the survey form distribution processing unit 14 determines whether survey form information distribution to all clinical trial target patients is ended. As the result of the determination, if a patient to whom the survey form information is not distributed yet remains, the survey form distribution processing unit 14 returns to step S16 to perform the distribution processing of the survey form information to the next patient terminal UT. Similarly, the survey form distribution processing unit 14 repetitively executes the distribution processing of the survey form information to each clinical trial target patient until distribution of all clinical trial target patients is ended.

Note that as the survey form distribution procedure, another procedure is also possible. For example, the server apparatus SV first transmits a survey request message to the patient terminal UT using email, SMS, or SNS. The survey request message includes a URL indicating a survey answer page. If the patient performs an access operation to the URL on the patient terminal UT, the survey form information is downloaded from the server apparatus SV to the patient terminal UT.

### (5) Acquisition and Transfer of Patient Reported Outcome Information

If the survey form information is distributed from the server apparatus SV, the survey form information is displayed on the display screen of the patient terminal UT. If the patient US inputs a state after taking the medicine, that is, answer information indicating a prognosis to each survey item of the survey form information in this state, the input answer information is uploaded as patient reported outcome information to the server apparatus SV. Note that the operation of inputting the answer information is performed by, for example, inputting a checkmark to the checkbox of an answer candidate corresponding to the state of the patient himself/herself among a plurality of answer candidates described in each answer field of the survey form information.

If distribution of the survey form information is ended, as shown in FIG. 5, the control unit 1 of the server apparatus SV monitors reception of patient reported outcome information in step S20 under the control of the patient reported outcome reception processing unit 15. If patient reported outcome information is sent from the patient terminal UT in this state, in step S21, the patient reported outcome reception processing unit 15 receives, via the communication I/F 4, the patient reported outcome information sent from the patient terminal UT, and stores the received patient reported outcome information in the patient reported outcome information storage unit 35 together with the clinical trial ID.

Then, in step S22, the patient reported outcome reception processing unit 15 determines whether reception of patient reported outcome information from all clinical trial target patients is completed. If a patient whose patient reported outcome information is not received yet remains, the patient reported outcome reception processing unit 15 returns to step S20 to continue patient reported outcome information reception and storage processing in steps S20 and S21.

Also, in parallel to the patient reported outcome information reception processing, the control unit 1 of the server apparatus SV executes processing of transferring the patient reported outcome information to the information processing system of the pharmaceutical company PS in the following way under the control of the patient reported outcome transfer processing unit 16.

That is, first, in step S23, the patient reported outcome transfer processing unit 16 determines whether the patient reported outcome information acquisition state satisfies a predetermined transfer condition. For example, the patient reported outcome transfer processing unit 16 determines whether the number of untransferred patient reported outcome information stored in the patient reported outcome information storage unit 35 reaches a predetermined number. As the result of the determination, if the number of untransferred patient reported outcome information reaches a predetermined number, in step S24, the patient reported outcome transfer processing unit 16 reads out the untransferred patient reported outcome information from the patient reported outcome information storage unit 35, and transmits the readout patient reported outcome information from the communication I/F 4 to the information processing system of the pharmaceutical company PS of the clinical trial request source.

Note that as for the patient reported outcome information transfer processing, patient reported outcome information may individually be transferred every time a piece of patient reported outcome information is received. Alternatively, every time a preset date/time elapses, pieces of patient reported outcome information stored during the period may be transferred at once.

In step S25, while performing the reception processing and the transfer processing of the patient reported outcome information, the control unit 1 of the server apparatus SV determines whether a clinical trial period designated by the clinical trial condition has elapsed. As the result of the determination, if the clinical trial period has not elapsed yet, the control unit 1 returns to step S20 to repetitively perform the above-described reception processing of the patient reported outcome information and the transfer processing of the received patient reported outcome information. On the other hand, if the clinical trial period has elapsed, in step S26, the control unit 1 of the server apparatus SV transfers, at once, untransferred patient reported outcome information among the patient reported outcome information stored in the patient reported outcome information storage unit 35.

Note that in the above-described patient reported outcome information reception processing and transfer processing, the patient reported outcome reception processing unit 15 may transmit a demand message to the patient terminal UT that has not returned patient reported outcome information when a predetermined time elapses from the survey form information transmission or every time a predetermined time elapses. Also, for example, a "consent revoke button" may be included in the demand message, and if the patient operates the "consent revoke button", the attribute information of the patient may be deleted from the clinical trial object person information storage unit 32.

### (Detailed Example)

### (1) Setting Sheet That Defines Survey Items

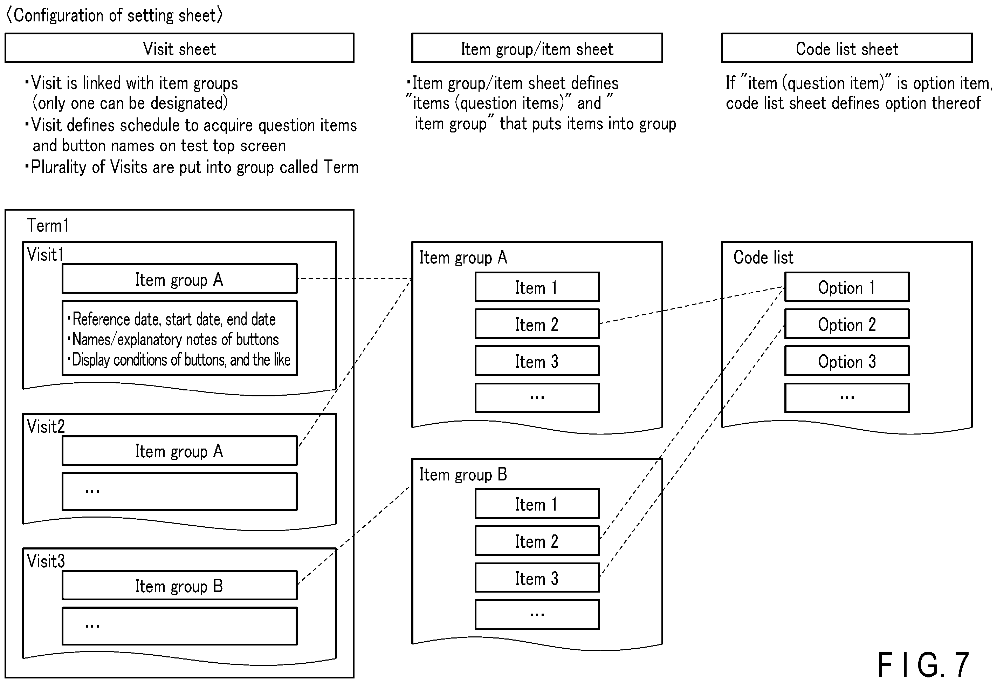

FIG. 7 is a view showing an example of the configuration of a setting sheet that defines survey items.

The setting sheet is formed by a Visit sheet, an item group/item sheet, and a code list sheet.

The item group/item sheet defines question items and item groups each puts a plurality of question items into a group. If a question item is an option item, the code list sheet is used to define the option.

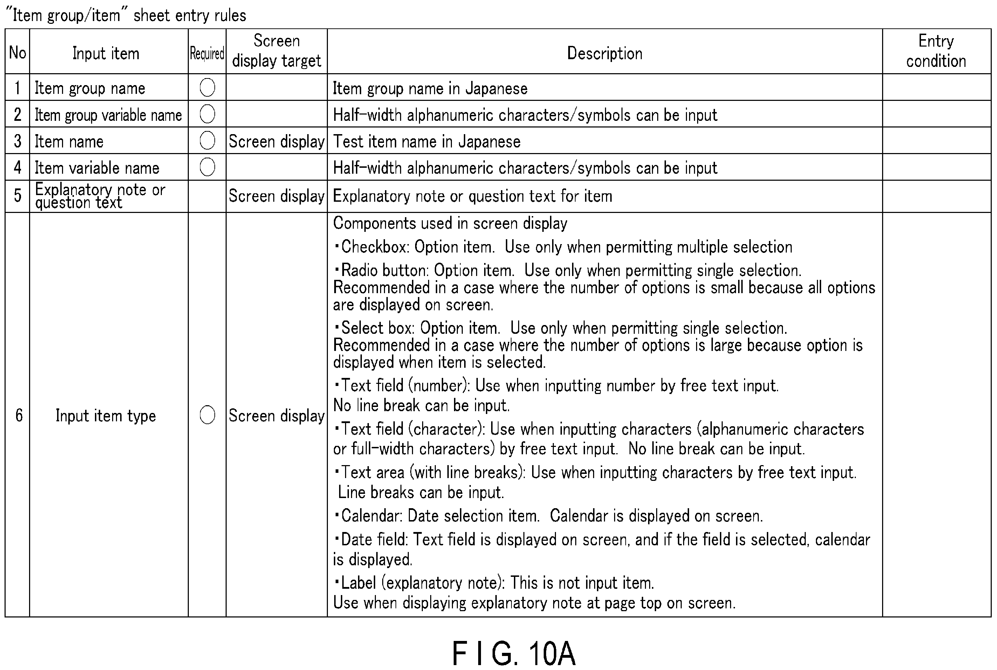

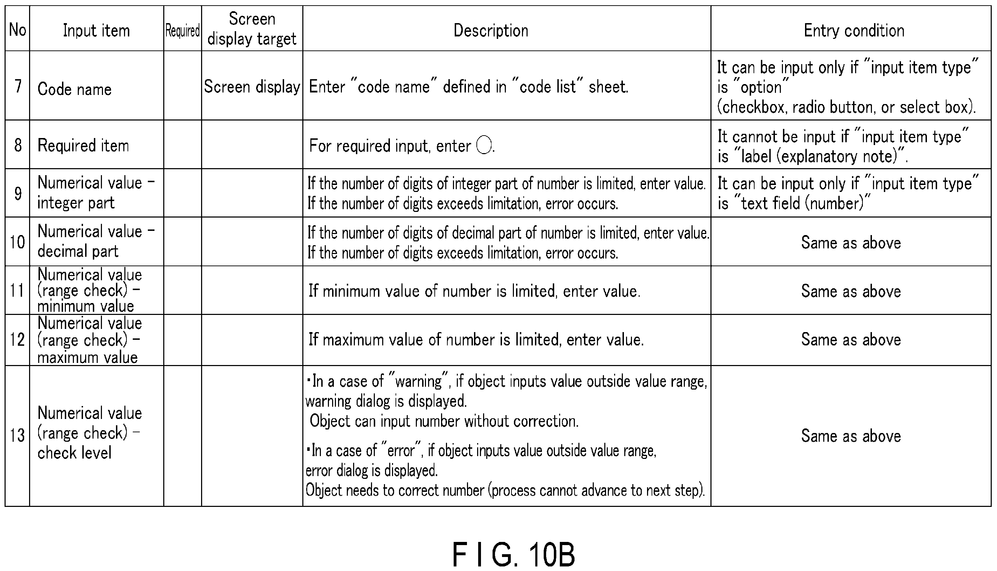

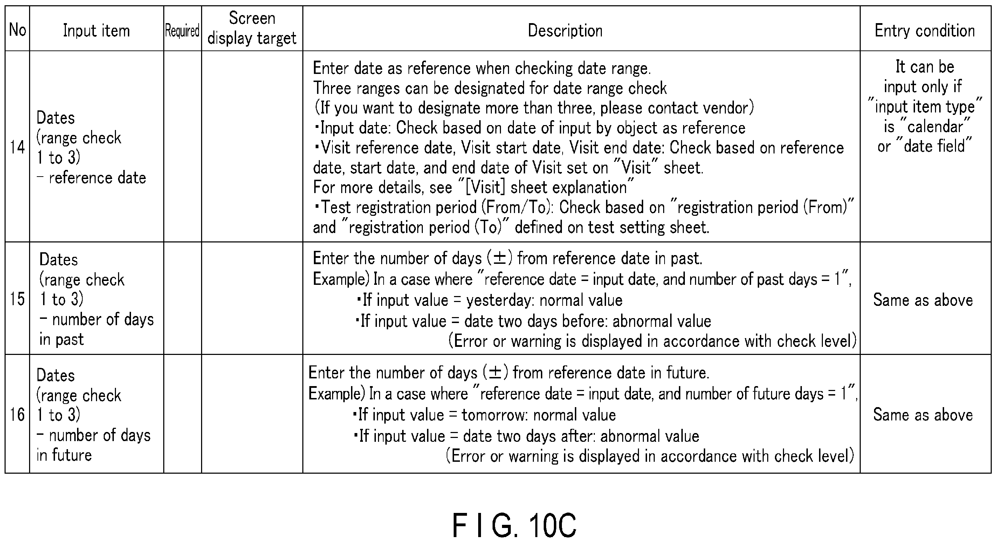

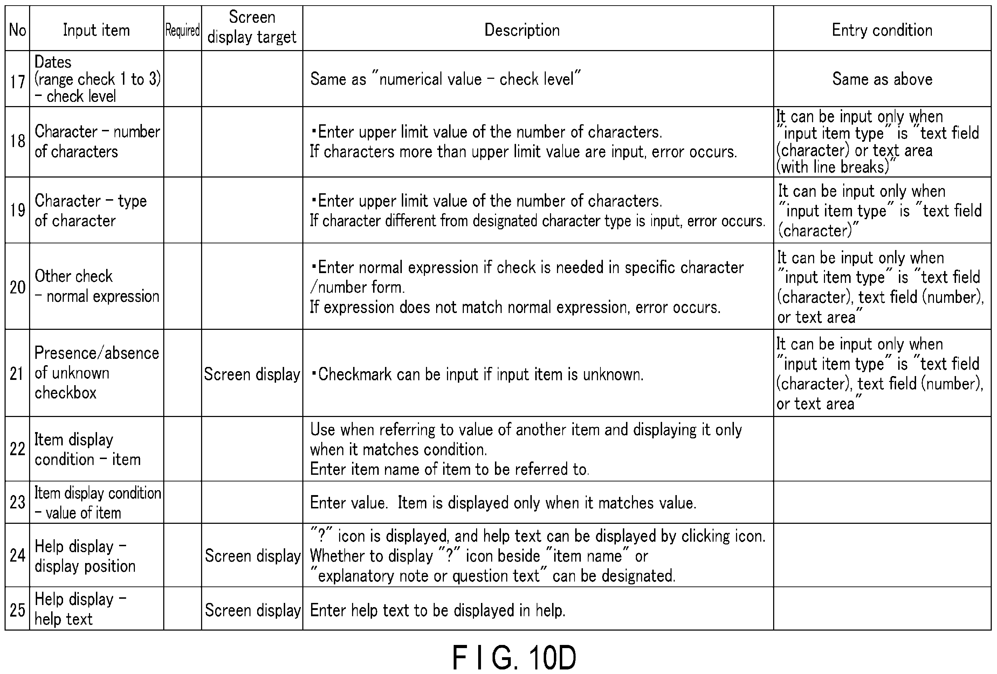

FIG. 10A shows an example of the item group/item sheet and entry rules thereof. FIG. 10B shows an example of the item group/item sheet and entry rules thereof, continued from FIG. 10A. FIG. 10C shows an example of the item group/item sheet and entry rules thereof, continued from FIG. 10B. FIG. 10D shows an example of the item group/item sheet and entry rules thereof, continued from FIG. 10C. As shown in FIGS. 10A, 10B, 10C, and 10D, in the item group/item sheet, attributes are defined for 25 items from "item group name" to "help display".

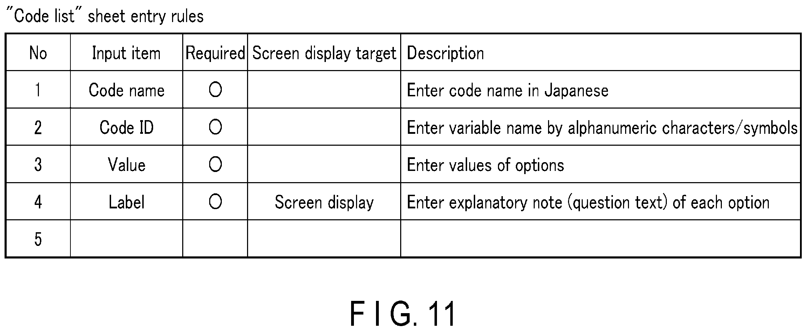

FIG. 11 shows an example of the configuration and entry rules of the code list sheet. In the code list sheet, a code name, the value of a code ID option, and a label describing the explanatory note (question text) of the option are defined.

The Visit sheet is linked with the item groups, and defines a schedule to acquire an answer to a question item from an object and a type button name to be displayed on a test top screen. Visit sheets include a Visit sheet generated for each Visit and a Visit sheet generated for each term that puts a plurality of Visits into a group.

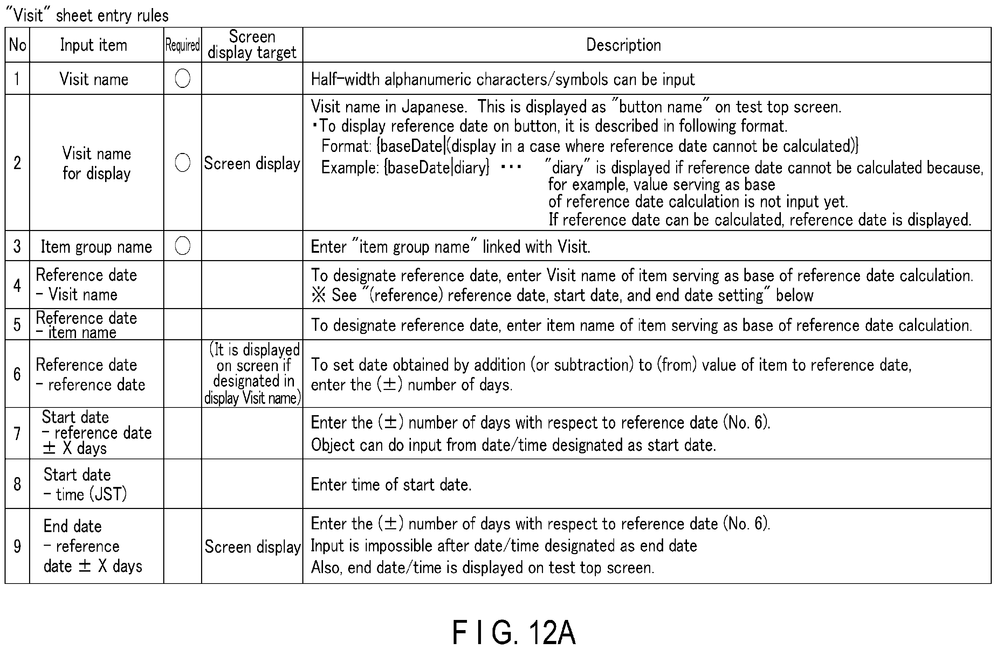

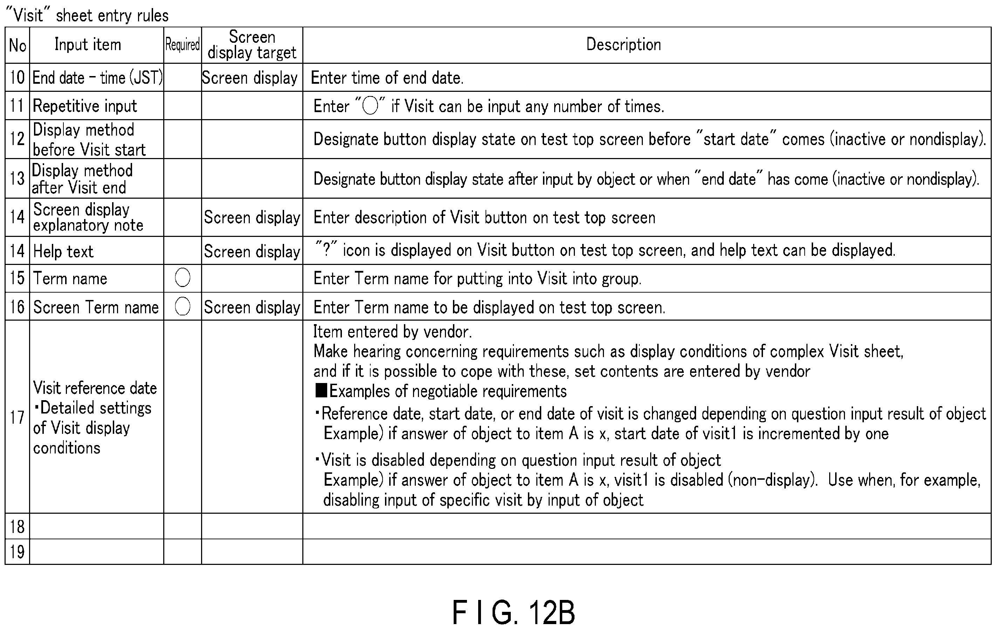

FIG. 12A shows an example of the configuration of the Visit sheet and entry rules thereof. FIG. 12B shows an example of the configuration of the Visit sheet and entry rules thereof, continued from FIG. 12A. In the Visit sheet, attributes are defined for 17 items from "Visit name" to "detailed settings of Visit reference date/Visit display condition" as input items. Note that the Visit reference date is used to set an answer input enable period (the start date and the end date) for an object, and designated to a date after an arbitrary number of days from a date input by the object.

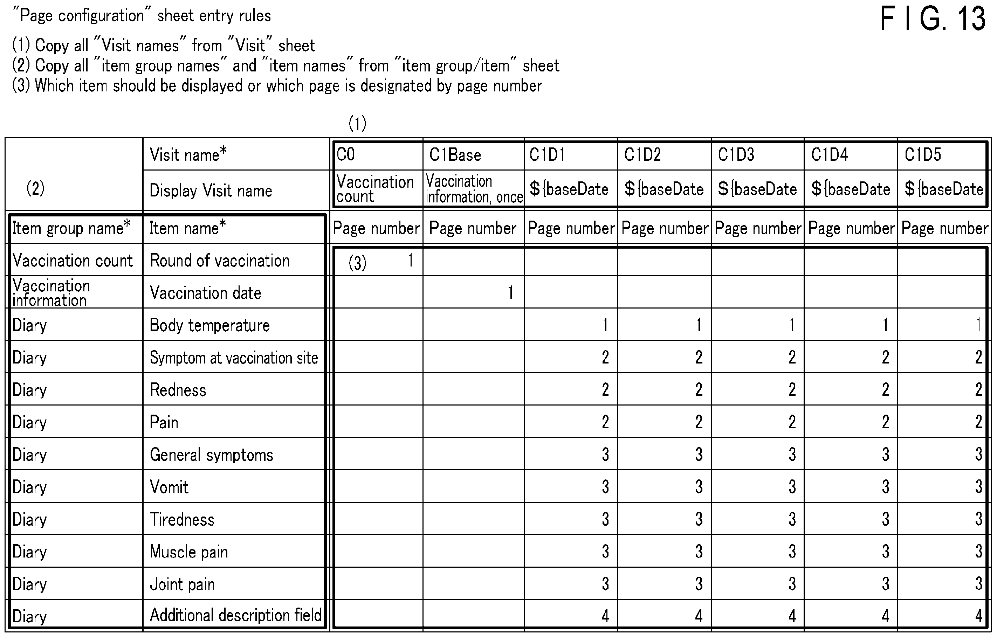

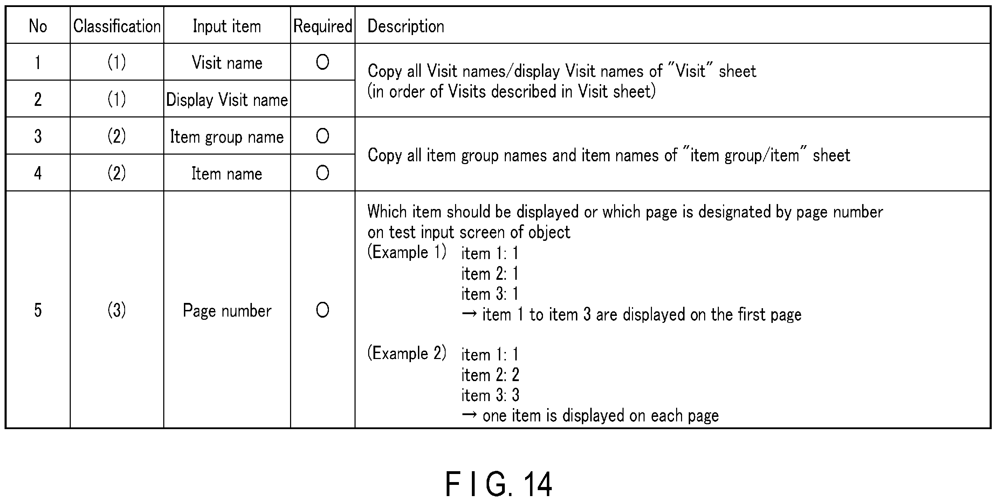

A page configuration sheet defines the page configuration of question items in each Visit, and is created by copying necessary items from the Visit sheet and the item group/item sheet. FIG. 13 shows an example of the configuration and entry rules of the page configuration sheet, and which question item should be displayed on which page is designated by a page number. FIG. 14 is a view for explaining input items in the page configuration sheet.

### (2) Test Input Screen

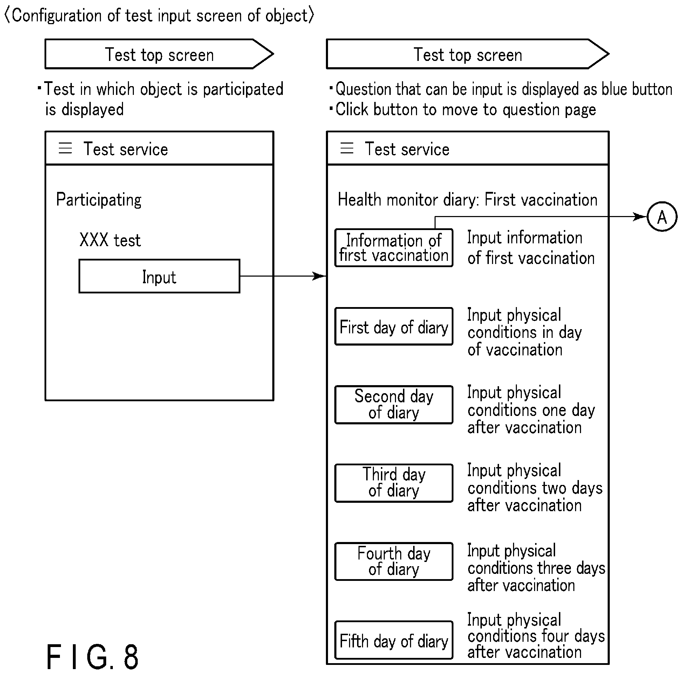

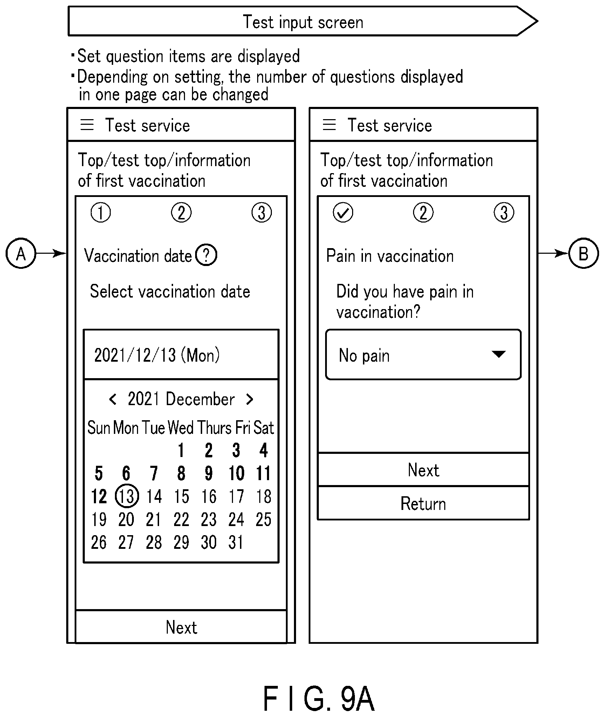

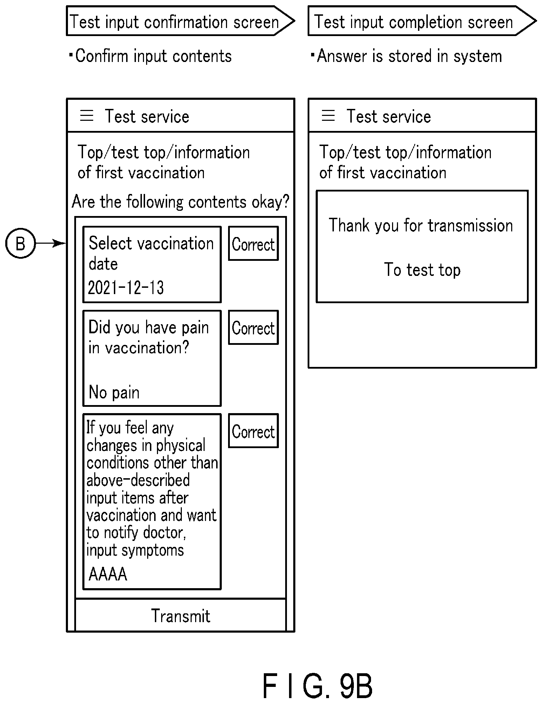

FIGS. 8, 9A, and 9B are views showing an example of the configuration of a test input screen generated based on the setting sheet shown in FIG. 7. Note that in this example, exemplifying vaccination, a description will be made in which a clinical trial will be referred to as a test.

A test input screen for an object is layered into a "test top screen", a "test input screen", a "test input confirmation screen", and a "test input completion screen". The test top screen is formed by an "initial screen" and a "date selection screen" to which transition occurs by a click operation on an input button displayed on the initial screen. On the date selection screen, a plurality of buttons are displayed to select a plurality of dates after a vaccination date, which are subjected to the test.

The test input screen transitions in accordance with a click operation of the date selection button. The test input screen includes a plurality of question pages. The question pages include a "vaccination count selection screen", a "vaccination date input screen", a "screen to input presence/absence of pain in vaccination", a "screen to input redness vanishment date", a "body temperature input screen", a "screen to select and input symptom at vaccination site", a "screen to input physical conditions after vaccination", and a "screen to input message".

The test input confirmation screen is a screen that displays a list of answers input in each question page of the test input screen, and a correction button is displayed in correspondence with each display field provided for each answer. If a click operation is performed on the correction button, display returns to a corresponding question page to enable correction input of the answer. Also, a transmission button is displayed at the end of the test input confirmation screen. If a click operation is performed on the transmission button, answer data transmission processing is executed, and the screen transitions to the test input completion screen.

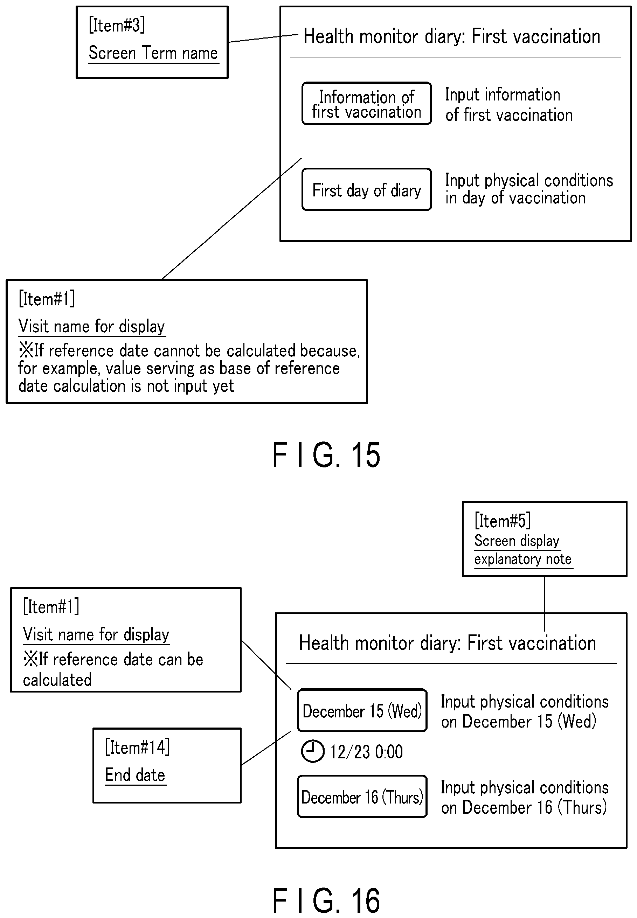

FIGS. 15, 16, 17, 18, 19, 20, 21, 22, 23, and 24 show an example of the association between the test input screen and the items defined by the item group/item sheet.

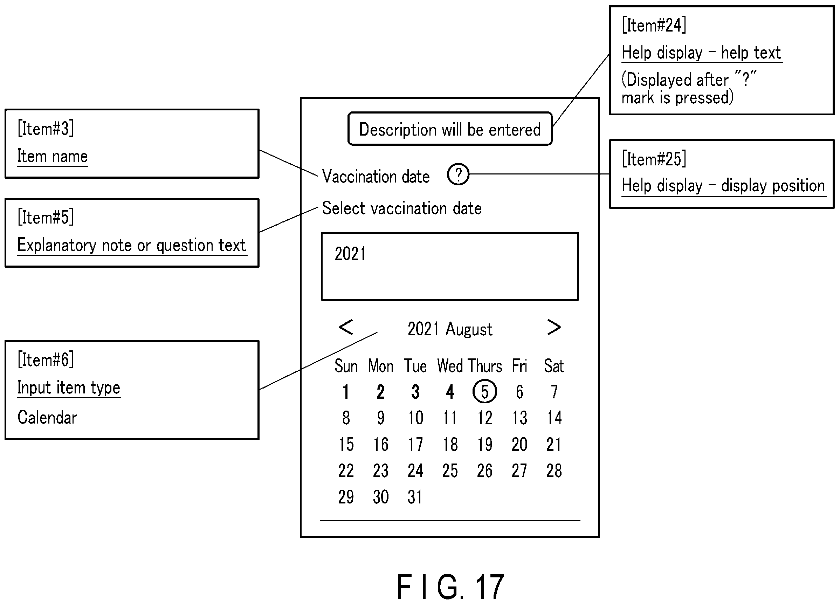

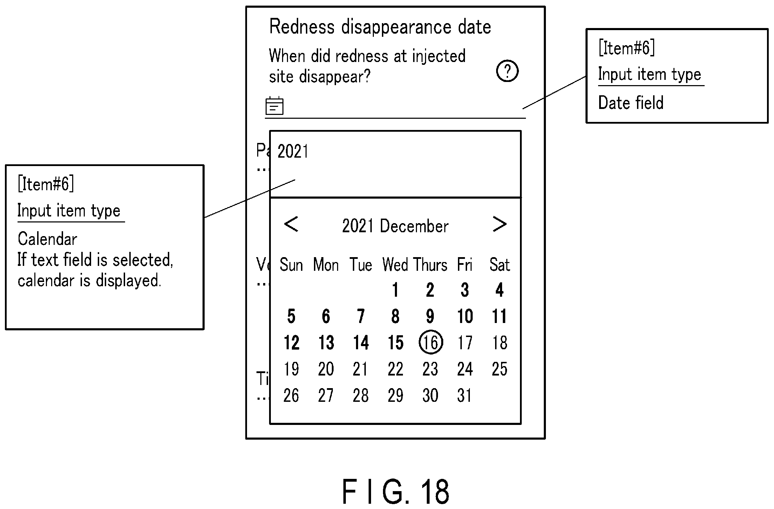

For example, on the "date selection screen" shown in FIG. 15, item group name (display Visit name) #1 and screen term name #3 are reflected. On the "physical condition input screen" shown in FIG. 16, item group name (display Visit name) #1, screen display explanatory note #5, and end date #14 are reflected. On the "vaccination date input screen" shown in FIG. 17, item name #3, explanatory note or question text #5, input item type #6, help display/display position #24, and help display/help text #25 are reflected. On the "screen to input redness vanishment date" shown in FIG. 18, a date field and a calendar defined by input item type #6 are reflected.

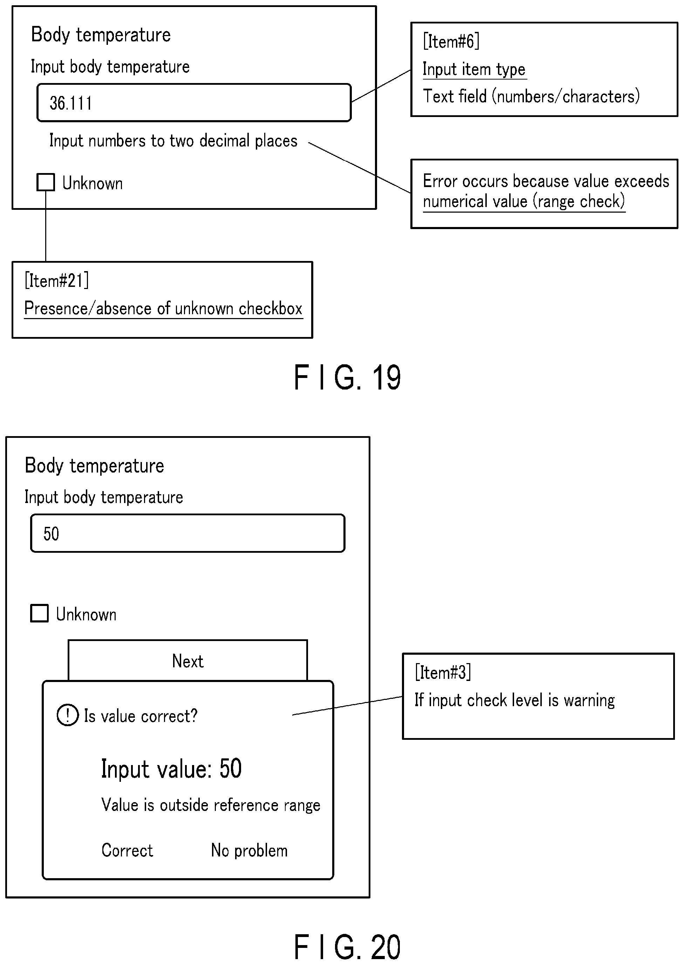

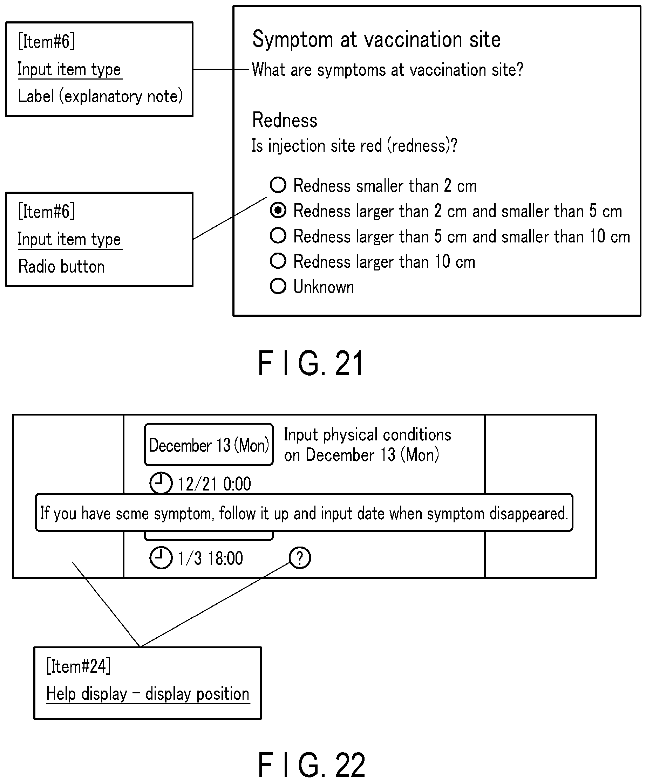

Also, for example, on the "body temperature input screen" shown in FIG. 19, input item type (text field) #6, and presence/absence of unknown checkbox #21 are reflected. On the "body temperature input confirmation screen" shown in FIG. 20, item name #3 is reflected. On the "screen to select and input symptom at vaccination site" shown in FIG. 21, a label and a radio button defined by input item type #6 are reflected.

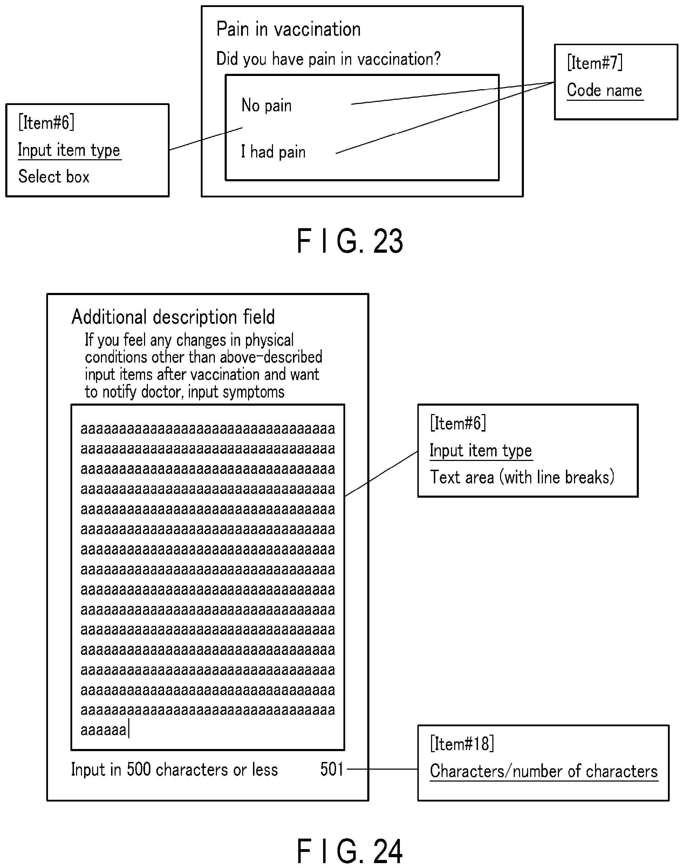

Also, for example, on the "physical condition input screen" shown in FIG. 22, help display/display position #24 is reflected. On the "screen to input presence/absence of pain in vaccination" shown in FIG. 23, a select box defined by input item type #6 and code name #7 are reflected. On the "screen to input additional description field" shown in FIG. 24, a text area defined by input item type #6 and character/character count #18 are reflected.

### (3) Display Examples of Test Input Screens

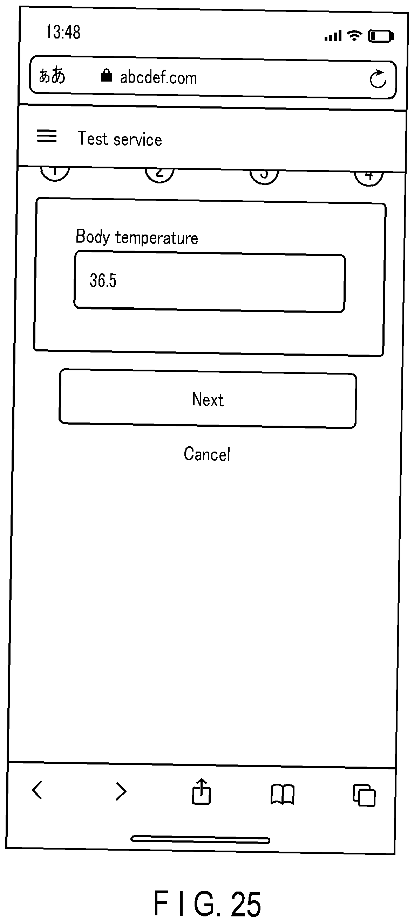

FIGS. 25, 26, 27, 28, and 29 show examples of display of the test input screens generated by reflecting the items as described above, on an object terminal.

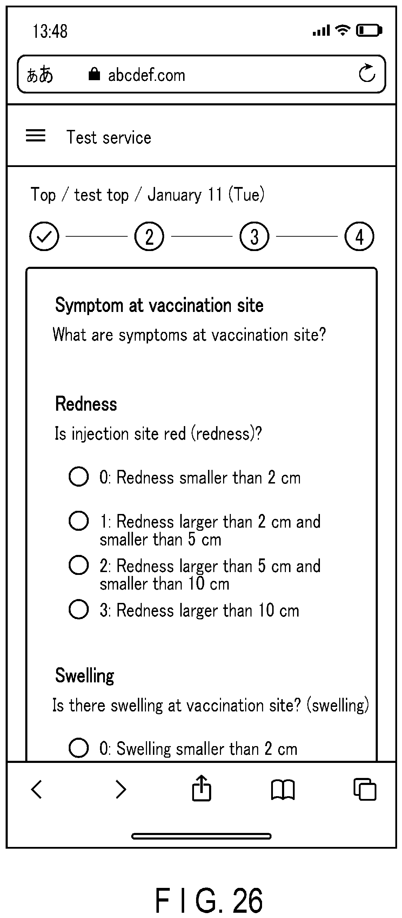

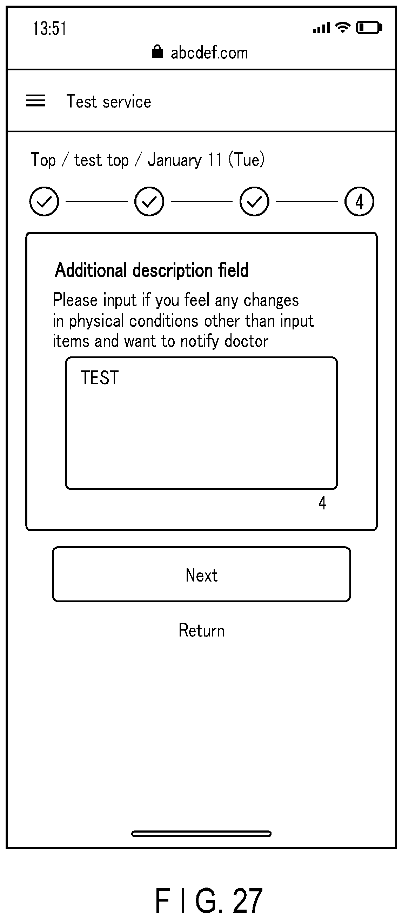

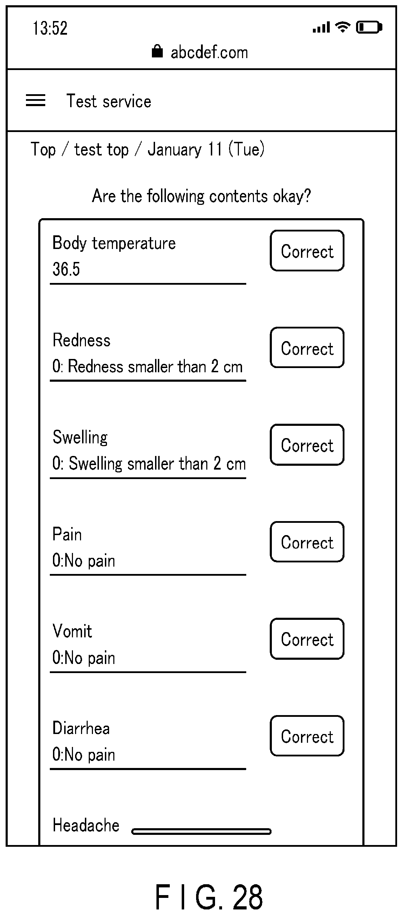

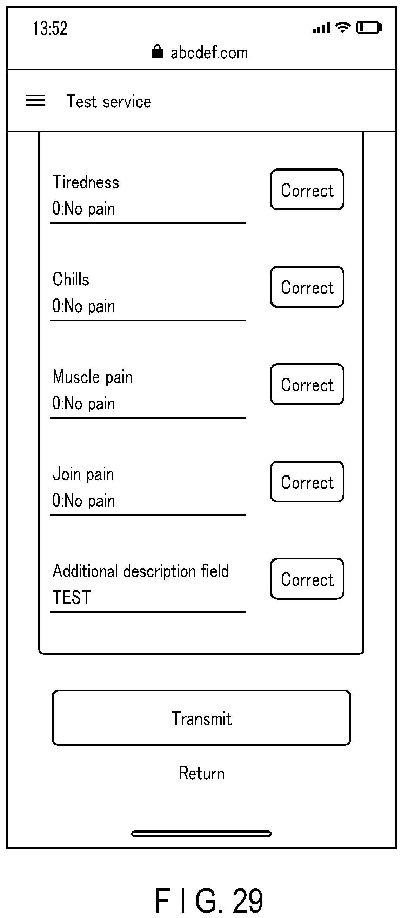

FIG. 25 shows a display example of the "physical condition input screen", FIG. 26 shows a display example of the "screen to input symptom at vaccination site", and FIG. 27 shows a display example of the "screen to input additional description field". Also, FIGS. 28 and 29 show display examples of the "test input confirmation screen".

### (Effects)

As described above, in an embodiment, in the server apparatus SV, clinical trial protocol information is acquired from a pharmaceutical company in accordance with a clinical trial cooperation request from the pharmaceutical company, and a patient who satisfies clinical trial conditions included in the clinical trial protocol information is selected as a clinical trial target patient. In addition, list data of survey items included in the clinical trial protocol information is imported using conversion rules stored in advance and edited into a predetermined format, thereby generating survey form information for each clinical trial. The generated survey form information is transmitted to the patient terminal UT of the selected clinical trial target patient, and an answer result input to the survey form information is received as patient reported outcome information from the patient terminal UT and transferred to the pharmaceutical company PS of the clinical trial request source.

Hence, a series of processes from creation of survey form information for a clinical trial of a medicine to collection of patient reported outcome information of the patient is automatically performed by the server apparatus SV. For this reason, the clinical trial manager of the pharmaceutical company PS need not create survey form information by himself/herself for each clinical trial, and the work of selecting clinical trial target patients and the work of distributing survey form information to each patient and collecting patient reported outcome information are unnecessary. This can reduce the work load concerning the clinical trial, and shorten time needed for the clinical trial and increase the efficiency of the clinical trial.

### [Other Embodiments]

(1) In the embodiment, an example in which the server apparatus SV directly distributes the clinical trial cooperation request and the clinical trial protocol information to the patient terminal UT, and acquires patient reported outcome information input by the patient from the patient terminal UT has been described. However, there also exists a patient who does not have a terminal of his/her own, or even if it does, the patient may have difficulty in inputting a patient reported outcome because of inexperience with the operation.

For such a patient, family living together, or the medical institution MS or the pharmacy DS may report by making an interview with the patient. For example, if a patient has difficulty in reporting by himself/herself, the contact information of a terminal of family living together or a terminal of a medical institution or pharmacy for the family is included in the attribute information of the patient. Based on the contact information included in the attribute information of the patient, the server apparatus SV distributes the clinical trial cooperation request and the clinical trial protocol information to the terminal. Then, the family living together, or a person in change in the medical institution or the pharmacy obtains the prognosis state from the patient by an interview directly or using a telephone, and transmits the result of interview as patient reported outcome information to the server apparatus SV.

(2) The server apparatus SV determines whether the patient reported outcome information sent from the patient terminal UT includes an answer result indicating a suspicious harmful phenomenon by a medicine, such as a serious side effect caused by vaccination or a serious side effect caused by administration of another medicine. This determination is performed based on, for example, the position of a checkbox with a checkmark in an answer field. As the result of the determination, if an answer result indicating a suspicious harmful phenomenon is included, the server apparatus SV may not only transfer the patient reported outcome information to the information processing system of the pharmaceutical company PS but also transmit the patient reported outcome information to the information processing system of the medical institution MS as well via the network.

This allows, for example, the medical institution to early conduct online diagnosis for the patient. However, to enable the above-described processing, when selecting the clinical trial target patient, personal information including the name of the patient needs to be acquired. For this reason, at the time of clinical trial target patient selection processing, or at the time of consent confirmation to the clinical trial in the medical institution or the pharmacy before that, it is necessary to obtain, from the patient, a consent concerning acquisition of personal information including the name.

(3) For the configuration and processing procedure of the patient reported outcome collection support apparatus, the clinical trial target patient selection method, the survey form information generation method, the survey form information distribution method, the types and the number of survey items, the survey contents, and the display format of survey form information as well, various changes and modifications can be made without departing from the scope of the present invention.

A program according to this embodiment, that is, a program to be executed by the processor of the patient reported outcome collection support apparatus or the like may be stored in an electronic device such as the patient reported outcome collection support apparatus and transferred in this state, or may be transferred without being stored in an electronic device. In the latter case, the program may be transferred via the network, or may be stored in a storage medium and transferred in this state. The storage medium is a non-transitory concrete medium. The storage medium is a computer-readable medium. The storage medium need only be a computer-readable medium capable of storing the program, such as a CD-ROM or a memory card, and its form is not particularly limited.

The embodiments of the present invention have been described above in detail, but the above description is merely an example of the present invention in all aspects. It is possible to make various changes and modifications without departing from the scope of the present invention, as a matter of course. That is, when implementing the present invention, a detailed configuration according to the embodiment may appropriately be adopted.

That is, the present invention is not limited to the above-described embodiments, and the constituent elements can be modified and embodied without departing from the scope of the present invention at the stage of implementation. Also, various inventions can be formed by appropriately combining a plurality of constituent elements disclosed in the embodiments. For example, some constituent elements may be removed from all constituent elements disclosed in the embodiments. Also, constituent elements according to different embodiments may appropriately be combined.

### REFERENCE SIGNS LIST

SV... server apparatus
UT1 to UTn, UT...patient terminal
MS1 to MSj, MS...medical institution
PS1 to PSk, PS...pharmaceutical company
DS1 to DSi, DS._.pharmacy
NW... network
1...control unit
2...program storage unit
3...data storage unit
4... communication I/F
5...bus
11...clinical trial protocol information acquisition processing unit
12...clinical trial object person selection processing unit
13...survey form generation processing unit
14...survey form distribution processing unit
15...patient reported outcome reception processing unit
16...patient reported outcome transfer processing unit
31... clinical trial protocol information storage unit
32...clinical trial object person information storage unit
33...conversion rule storage unit
34...survey form information storage unit
35...patient reported outcome information storage unit

## Claims

1. A patient reported outcome collection support apparatus capable of transmitting, via a network, information data between an information processing apparatus used by a clinical trial operator of a medicine and each of a plurality of terminal apparatuses used by a plurality of patients or substitutes thereof, comprising:
an acquisition processing unit configured to acquire clinical trial protocol information including a plurality of survey items defined for each clinical trial in advance from the information processing apparatus via the network;
a generation processing unit configured to generate survey form information used to survey a prognosis of the patient by converting the plurality of survey items included in the acquired clinical trial protocol information based on a conversion rule stored in advance;
a patient reported outcome acquisition processing unit configured to distribute the generated survey form information to the terminal apparatus via the network and acquire patient reported outcome information indicating an answer result of the patient to the survey form information from the terminal apparatus via the network; and
a transfer processing unit configured to transfer the acquired patient reported outcome information to the information processing apparatus used by the clinical trial operator via the network.

2. The patient reported outcome collection support apparatus according to claim 1, wherein
the acquisition processing unit further acquires information indicating a clinical trial condition from the information processing apparatus, and
the patient reported outcome collection support apparatus further comprises a clinical trial object person selection processing unit configured to select a clinical trial target patient satisfying the acquired clinical trial condition from the plurality of patients by referring to attribute information, medical treatment information, and prescription information of the plurality of patients.

3. The patient reported outcome collection support apparatus according to claim 1, wherein the generation processing unit converts, in accordance with the conversion rule, data of the survey item created by a first application into data that can be handled by a second application, and the second application generates the survey form information.

4. The patient reported outcome collection support apparatus according to claim 3, wherein the generation processing unit converts the data of the survey item created in a table format by the first application into data of a document format that can be handled by the second application, and the second application generates the survey form information of the document format including question information corresponding to the survey item and answer candidate information therefor.

5. The patient reported outcome collection support apparatus according to claim 1, further comprising a transmission processing unit configured to determine whether the acquired patient reported outcome information includes an answer result indicating a suspicious harmful phenomenon by the medicine, and if the answer result indicating the suspicious harmful phenomenon is included, transmit the patient reported outcome information to the information processing apparatus used by a medical institution via the network.

6. A patient reported outcome collection support method executed by a support information processing apparatus capable of transmitting, via a network, information data between an information processing apparatus used by a clinical trial operator of a medicine and each of a plurality of terminal apparatuses used by a plurality of patients, comprising:
acquiring clinical trial protocol information including a plurality of survey items defined for each clinical trial in advance from the information processing apparatus used by the clinical trial operator via the network;
generating survey form information used to survey a prognosis of the patient by converting the plurality of survey items included in the acquired clinical trial protocol information based on a conversion rule stored in advance;
distributing the generated survey form information to the terminal apparatus via the network and acquiring patient reported outcome information indicating an answer result of the patient to the survey form information from the terminal apparatus via the network; and
transferring the acquired patient reported outcome information to the information processing apparatus used by the clinical trial operator via the network.

7. A program configured to cause a processor provided in a patient reported outcome collection support apparatus defined in claim 1 to execute processing executed by at least one of processing units included in the patient reported outcome collection support apparatus.

8. A non-transitory computer-readable storage medium storing a program executed by a processor of a support information processing apparatus capable of transmitting, via a network, information data between an information processing apparatus used by a clinical trial operator of a medicine and each of a plurality of terminal apparatuses used by a plurality of patients, causing the processor to:
acquire clinical trial protocol information including a plurality of survey items defined for each clinical trial in advance from the information processing apparatus used by the clinical trial operator via the network;
generate survey form information used to survey a prognosis of the patient by converting the plurality of survey items included in the acquired clinical trial protocol information based on a conversion rule stored in advance;
distribute the generated survey form information to the terminal apparatus via the network and acquire patient reported outcome information indicating an answer result of the patient to the survey form information from the terminal apparatus via the network; and
transfer the acquired patient reported outcome information to the information processing apparatus used by the clinical trial operator via the network.
